# EUROPÄISCHE PATENTSCHRIFT

(11) **EP 1 474 055 B1**
(45) Veröffentlichungstag und Bekanntmachung des Hinweises auf die Patenterteilung: **03.05.2006**
(21) Anmeldenummer: 02710743.2
(22) Anmeldetag: 15.02.2002
(51) Int. Cl.: A61B 17/80

(54) **KNOCHENPLATTE**
BONE PLATE
LAME OSSEUSE

(43) Veröffentlichungstag der Anmeldung: 10.11.2004
(73) Patentinhaber: Medartis AG, 4051 Basel (CH)
(72) Erfinder: PFEFFERLE, Joachim, 79244 Münstertal (DE); BLUME, Oliver, 80331 München (DE); ZEUNER, Hermann, D-79111 Freiburg (DE)
(74) Vertreter: Heinen, Detlef
(86) Internationale Anmeldenummer: PCT/CH2002/000094
(87) Internationale Veröffentlichungsnummer: WO 2003/068091

(56) Entgegenhaltungen:
- WO-A-00/66012
- WO-A-00/71031
- US-A- 5 822 865
- US-A- 6 001 099
- US-A- 6 053 919

## Beschreibung

Die Erfindung betrifft eine Knochenplatte zur Versorgung einer Fraktur gemäss dem Oberbegriff des unabhängigen Patentanspruchs.

Bei der Versorgung von Frakturen insbesondere des atrophischen Unterkiefers, die bei Patienten höheren Alters in zunehmendem Masse auftreten, können zahlreiche Probleme auftreten. So stellen beispielsweise lokale Faktoren wie ungenügende Durchblutung durch das häufige Fehlen der Zentralarterie und sklerotische kompaktäre Knochenstümpfe ein Risiko für die Konsolidierung dar. Ausserdem bietet der reduzierte Knochenquerschnitt nicht nur eine geringe Anlagerungsfläche der Enden der Knochenfragmente diesseits und jenseits der Fraktur, sondern erschwert auch das anatomisch korrekte Reponieren der Knochenfragmente. Bei sehr starken Atrophien kann der frakturnahe Knochen so dünn sein, dass grösser dimensionierte Osteosyntheseschrauben von stärkeren Systemen (z.B. von sogenannten mandibularen Trauma- und Rekonstruktionsplatten) nur frakturfern stabil verankert werden können. Eine Kompression der Fraktur ist somit nicht möglich. Bei zahnlosen Patienten ist zudem das Anbringen von Schienenverbänden praktisch nicht möglich. Konservative Therapiemethoden vermögen nur schwer den permanenten suprahyoidalen Muskelzug auf die zahnlosen Fragmente zu kompensieren. Darüberhinaus können auch Erkrankungen, die im fortgeschrittenen Alter vermehrt auftreten, wie z.B. Osteoporose, Diabetes, Niereninsuffizienz und viele andere den.Heilungsverlauf ungünstig beeinflussen. Daneben limitieren im hohen Alter der Allgemeinzustand der Patienten und das hohe Morbiditätsrisiko bei einer längeren Immobilisierung den Umfang und die Dauer operativer Eingriffe.

Bisher werden verschiedene Therapieansätze verfolgt. Dabei spielt die konservative, nicht-invasive Behandlungsmethode inzwischen eine weniger gewichtige Rolle. Zwei wichtige Ansätze betreffen die Versorgung der Fraktur entweder mit einer sogenannten Miniplatte oder mit einer sogenannten Rekonstruktionsplatte.

Bei Miniplatten handelt es sich um Knochenplatten mit einer relativ geringen Materialstärke. Solche Miniplatten haben den Vorteil, dass sie bei dem operativen Eingriff oral (also durch den Mund hindurch) eingebracht werden können, es ist also kein Hautschnitt im Kieferbereich des Patienten erforderlich. Die Miniplatten lassen sich gut an die Knochenfragmente anbiegen, aber es ist - wenn überhaupt - nur in wenigen Fällen eine Kompression der Knochenfragmente im Bereich der Fraktur möglich. Allerdings weisen Miniplatten - bedingt durch ihre Materialstärke - in Bezug auf die Stabilität der Immobilisierung der Knochenfragmente im Bereich der Fraktur gewisse Grenzen auf, vor allen Dingen im Hinblick darauf, dass die Patienten möglichst bald nach dem operativen Eingriff wieder kauen sollen und somit erhebliche Kräfte im Frakturbereich wirken.

Hier liegen die Vorteile der Rekonstruktionsplatten. Bei diesen Rekonstruktionsplatten handelt es sich um relativ massive Knochenplatten (Materialstärke typischerweise im Bereich von 2.0 - 3.0 mm), die sehr stabil sind und daher auch grosse Kräfte aufnehmen können. Rekonstruktionsplatten lassen sich aber nur schwer an die jeweilige Form des Unterkiefers des Patienten anbiegen. Rekonstruktionsplatten werden dementsprechend auch mit relativ massiven, grossen Schrauben befestigt, die durch entsprechende an der Knochenplatte ausgebildete Ösen hindurch in den Knochen geschraubt werden, was bei ungenügendem Knochenangebot aber nur frakturfern erfolgen kann. Somit ist eine Kompression der Knochenfragmente im Frakturbereich nicht möglich, und ausserdem sind die Rekonstruktionsplatten häufig so gross (da sie eben nur frakturfern befestigt werden können), dass sie einen Hautschnitt im Kieferbereich nötig machen, um das Anbringen der Rekonstruktionsplatte am Unterkiefer zu ermöglichen.

Ausgehend von den vorstehend geschilderten Nachteilen der existierenden Knochenplatten liegt der vorliegenden Erfindung die Aufgabe zugrunde, eine Knochenplatte vorzuschlagen, die einerseits in unmittelbarer Nähe zum Frakturspalt ein hohes Mass an Stabilität aufweist und die frakturfern leicht an den Knochen anbiegbar (adaptierbar) ist. Ferner soll die Knochenplatte von ihren Abmessungen her möglichst klein sein, um beim operativen Eingriff ein orales Einbringen der Knochenplatte zu ermöglichen und somit einen Hautschnitt zu vermeiden. Vorzugsweise soll auch eine Kompressionsosteosynthese möglich sein.

Diese Aufgabe wird durch eine Knochenplatte gelöst, wie sie durch die Merkmale des unabhängigen Patentanspruchs charakterisiert ist. Vorteilhafte Ausgestaltungen der erfindungsgemässen Knochenplatte ergeben sich aus den Merkmalen der abhängigen Patentansprüche.

Insbesondere weist die erfindungsgemässe Knochenplatte eine Plattenstärke auf, die im Bereich von 0.5 mm bis 1.6 mm liegt, und umfasst einen Stabilisierungsbereich, der über der Fraktur zu liegen bestimmt ist. Zu beiden Seiten dieses Stabilisierungsbereichs sind Ösen angeordnet, durch deren Löcher hindurch die Schrauben in die Knochenfragmente geschraubt werden können. Eine derartige Knochenplatte mit einem solchen Stabilisierungsbereich erlaubt einerseits eine stabile Immobilisierung im unmittelbaren Bereich der Fraktur, andererseits kann die Knochenplatte frakturfern gut an die Knochenfragmente angebogen (adaptiert) werden. Besonders bevorzugt liegt dabei die Plattenstärke im Bereich von 0.8 mm bis 1.2 mm. Bei solchen Knochenplatten ist es möglich, klein dimensionierte Schrauben zur Befestigung der Knochenplatte zu verwenden, welche auch bei geringem Knochenangebot noch eingeschraubt werden können.

Bei einem Ausführungsbeispiel der erfindungsgemässen Knochenplatte ist der Stabilisierungsbereich als Steg ausgebildet. Die Knochenplatte kann dabei so ausgebildet sein, dass das Widerstandsmoment des Stabilisierungsbereichs bzw. des Stegs gegen ein Verbiegen der Knochenplatte in der Plattenebene gleich gross oder grösser ist als das entsprechende Widerstandsmoment der Knochenplatte im Bereich zwischen den Ösen. Dadurch erhält der Stabilisierungsbereich seine stabilisierende Eigenschaft.

Die Länge des Stegs kann bis zu etwa sechs mal dem Abstand der Ösen voneinander betragen. Ein derartiger Steg ermöglicht die gute Stabilität der Knochenplatte im unmittelbaren Bereich um den Frakturspalt herum, erlaubt aber andererseits die gute Anbiegbarkeit der Platte in einem frakturferneren Bereich. Dennoch können die Abmessungen (insbesondere die Länge) der Knochenplatte so gehalten werden, dass die Platte beim operativen Eingriff oral zugeführt werden kann, sodass kein Hautschnitt erforderlich ist.

Bei einem vorteilhaften Ausführungsbeispiel der erfindungsgemässen Knochenplatte ist die Aussenkontur des Stegs oval. Diese Aussenkontur hat sich als besonders vorteilhaft im Hinblick auf die aufzunehmenden Belastungen erwiesen, aber es kommen durchaus auch andere Konturen (z.B. solche Konturen, die im Mittelbereich des Stegs parallel zur Plattenlängsachse verlaufen und dann linear bis hin zu einer geringern Breite am Rand des Stegs abnehmen, oder solche Konturen, die von der Mitte des Stegs direkt linear bis hin zu einer geringeren Breite am Rand des Stegs abnehmen) in Betracht. Auch rechteckförmige Konturen, bei denen Oalso der Steg praktisch bis zu seinen Randregionen hin eine einheitliche Breite aufweist, kommen beispielsweise in Betracht.

Bei einem vorteilhaften Ausführungsbeispiel der erfindungsgemässen Knochenplatte entspricht die Breite des Stegs im wesentlichen dem Aussendurchmesser der Ösen. Somit kann auch die Breite der Knochenplatte klein gehalten werden, was das Zuführen und Anbringen der Knochenplatte erleichtert, ohne dass dabei die Stabilität der Knochenplatte leidet.

Bei einem weiteren vorteilhaften Ausführungsbeispiel der erfindungsgemässen Knochenplatte nimmt die Breite des Stegs von der Mitte des Stegs ausgehend hin zu den Randregionen des Stegs ab. In der Mitte des Steges, die unmittelbar über dem Frakturspalt zu liegen bestimmt ist, ist also die Knochenplatte am breitesten. Dort soll sie auch die grössten Belastungen aufnehmen können. Nach aussen hin, also zu den Randregionen des Stegs hin, nimmt die Breite des Stegs ab, dort können die aufzunehmenden Belastungen auch schon wieder teilweise von den Knochenfragmenten aufgenommen werden ohne den Heilungsprozess im Bereich des Frakturspalts zu beeinträchtigen.

Bei einer vorteilhaften Ausbildung der erfindungsgemässen Knochenplatte können in den Randregionen des Stabilisierungsbereichs bzw. des Stegs Löcher vorgesehen sein, durch welche hindurch Schrauben in die Knochenfragmente geschraubt werden können. Die Knochenplatte kann dann schon relativ frakturnah - mit entsprechend weniger massiven Knochenschrauben - an den Knochenfragmenten befestigt werden. Die Löcher in den Randregionen des Stabilisierungsbereichs bzw. Stegs können insbesondere als Kompressionslöcher ausgebildet sein, oder es können zusätzlich zu herkömmlichen Durchgangslöchern in den Randregionen des Stabilisierungsbereichs bzw. Stegs auch noch separate Kompressionslöcher vorgesehen sein.

Schliesslich können in den Randregionen des Stabilisierungsbereichs bzw. des Stegs Schlitze vorgesehen sein, um das Anbiegen der Knochenplatte an die Knochenfragmente bereits in diesen Randregionen des Stabilisierungsbereichs bzw. des Stegs zu erleichtern.

Weiterhin kann in den Löchern der Ösen und/oder in den Löchern in den Randregionen des Stabilisierungsbereichs bzw. des Stegs eine Eingriffskontur vorgesehen sein zum winkelstabilen Verblocken der Knochenplatte mittels eines an der Schraube vorgesehenen Verblockungsgewindes, wobei die Eingriffskontur ein Einschrauben der Schraube unter verschiedenen Winkeln zulässt. Löcher mit solchen Eingriffskonturen sind beispielsweise aus der WO-A-00/66012 bekannt. Das Vorsehen einer solchen Eingriffskontur ist in mehrerer Hinsicht vorteilhaft. Zum einen sind keine Bohrlehren erforderlich, mit deren Hilfe Löcher für die Schrauben vorgebohrt werden müssen. Vielmehr kann das Vorbohren der Löcher ohne eine Bohrlehre erfolgen oder es können selbstbohrende Schrauben ohne eine Führung (also ohne Vorbohren) direkt in die Knochenfragmente eingeschraubt werden, weil die Eingriffskontur ein Einbringen der Schrauben unter verschiedenen Winkeln erlaubt. Gleichzeitig erlaubt die Eingriffskontur eine stabile Verblockung, sodass ein Selbstlösen der Schraube ausscheidet. Die Verbindung von Schraube und Platte ist grundsätzlich zwar wieder lösbar, jedoch ist hierzu ein deutlich höheres Drehmoment aufzuwenden als bei herkömmlichen Gewindeverbindungen.

Weitere vorteilhafte Ausgestaltungen ergeben sich aus der nachfolgenden Beschreibung von Äusführungsbeispielen der erfindungsgemässen Knochenplatte mit Hilfe der Zeichnung.

Es zeigen:
- Fig. 1: ein erstes Ausführungsbeispiel einer erfindungsgemässen Knochenplatte in Aufsicht,
- Fig. 2: einen Längsschnitt gemäss der Linie II-II aus Fig. 1 durch das Ausführungsbeispiel der Knochenplatte gemäss Fig. 1,
- Fig. 3: ein zweites Ausführungsbeispiel einer erfindungsgemässen Knochenplatte in Aufsicht
- und:
- Fig. 4: ein drittes Ausführungsbeispiel einer erfindungsgemässen Knochenplatte in Aufsicht.

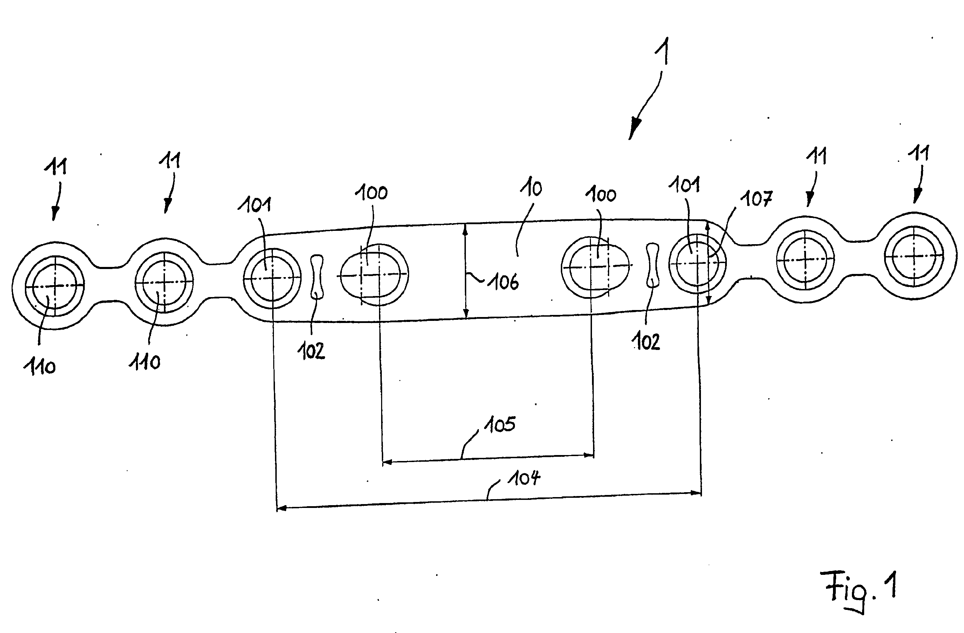

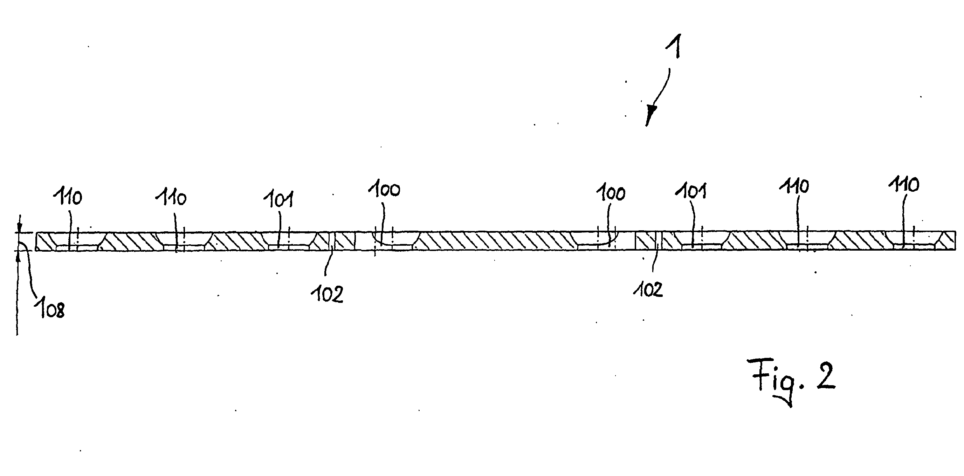

Das in Fig. 1 und Fig. 2 gezeigte erste Ausführungsbeispiel der erfindungsgemässen Knochenplatte 1 umfasst einen Stabilisierungsbereich, der hier als ein ovaler Steg 10 ausgebildet ist. Ausserhalb des Stegs 10 sind zu beiden Seiten Ösen 11 vorgesehen - bei dem hier gezeigten Ausführungsbeispiel jeweils zwei Ösen 11 (die ihrerseits durch einen schmalen Verbindungssteg mit dem als Stabilisierungsbereich wirkenden Steg 10 bzw. untereinander verbunden sind) - die zur Befestigung der Knochenplatte 1 im frakturfernen Bereich vorgesehen sind. Die Löcher 110 der Ösen 11 können entweder als Gewindelöcher ausgebildet sein, oder sie können so ausgebildet sein, dass sie - wie beispielsweise in der WO-A-00/66012 offenbart - eine Eingriffskontur zur winkelstabilen Verblockung aufweisen. Sie können auch gar kein Gewinde aufweisen, und die Befestigung der Knochenplatte erfolgt mit Hilfe von Knochenschrauben, die ausser dem Knochengewinde kein separates Gewinde aufweisen zum Eingriff in ein im Ösenloch 110 vorgesehenes Gewinde oder Eingriffskontur. Die Knochenplatte wird dann im Bereich der Öse 11 durch den Schraubenkopf an dem Knochenfragment fixiert.

Der Steg 10 weist in seinen Randregionen zunächst jeweils ein Kompressionsloch 100 auf. Die Art und Weise, wie solche Kompressionslöcher 100 ausgebildet sind sowie ihre Funktionsweise, sind an sich bekannt. Sie erlauben, die Enden der Knochenfragmente diesseits und jenseits des Frakturspalts zu komprimieren, was sich als förderlich für den Heilungsprozess erweisen kann (Kompressionsosteosynthese).

Weiterhin weist die in Fig. 1 gezeigte Knochenplatte 1 jeweils ein Durchgangsloch 101 auf, durch welches eine konventionelle Schraube hindurch geschraubt werden kann. Das Durchgangsloch 101 kann mit einem Gewinde oder mit einer Eingriffskontur, wie sie aus der WO-A-00/66012 bekannt ist, versehen sein, in welches ein entsprechendes Gewinde der Knochenschraube eingreift, es kann aber auch als ein Loch ohne Gewinde ausgebildet sein und es werden Knochenschrauben verwendet, die ausser dem Knochengewinde kein separates Gewinde aufweisen zum Eingriff in ein im Durchgangsloch 101 vorgesehenes Gewinde oder Eingriffskontur. Somit kann die Knochenplatte 1, gegebenenfalls nach erfolgter Kompression, schon relativ frakturnah an den Knochenfragmenten fixiert werden.

Zwischen dem jeweiligen Kompressionsloch 100 und dem unmittelbar benachbarten Durchgangloch 101 ist jeweils ein Schlitz 102 vorgesehen. Dieser Schlitz 102 bewirkt, dass die Knochenplatte schon in den Randregionen des Stegs 10 ein gewisses Anbiegen der Knochenplatte an die Knochenfragmente erlaubt. Allerdings ist die Knochenplatte in dem Bereich des Schlitzes 102 gegen ein Verbiegen der Knochenplatte in der Plattenebene immer noch stabiler (grösseres Widerstandsmoment) als im Bereich zwischen zwei Ösen 110 bzw. zwischen der unmittelbar benachbart zum Steg angeordneten Öse 110 und dem Steg 10.

Die Länge 104 des Stegs 10 (als solcher wird hier der Abstand 104 bezeichnet, weil der restliche Teil am Rande des Stegs 10 praktisch jeweils die Hälfte einer Öse darstellt) beträgt hier etwa das Vierfache des Abstands zweier benachbarter Ösenlöcher 110 - und damit des Abstands zweier Schrauben) voneinander. Die Kompressionslöcher 100 haben hier einen Abstand 105 voneinander, der zweimal dem Abstand zweier benachbarter Ösenlöcher 110 entspricht. Somit verbleibt ein genügend grosser Bereich des Stegs 10 zwischen den beiden Kompressionslöchern 100 zur stabilen Fixierung der Enden der Knochenfragmente, und dennoch bleiben die Abmessungen der Knochenplatte 1 insgesamt begrenzt.

Der Steg 10 weist insgesamt eine ovale Aussenkontur auf. Seine Breite nimmt von der Mitte her beginnend, wo er die Breite 106 aufweist, zu den Randregionen hin, wo er die Breite 107 aufweist, leicht ab, wodurch die Knochenplatte in den Randregionen des Stegs 10 bereits ein wenig leichter an die Knochenfragmente anbiegbar ist, aber dennoch eine ausreichende Stabilität im Hinblick auf die Immobilisierung im Bereich des Frakturspalts aufweist. Alternativ kommen auch andere Aussenkonturen (weiter oben schon erwähnt) in Betracht. Die Breite 106,107 des Stegs 10 stimmt hier im wesentlichen mit dem Aussendurchmesser der Ösen 11 überein, was einerseits eine ausreichende Stabilität der Knochenplatte 1 gewährleistet, andererseits ein einheitliches Plattendesign ermöglicht, was vom herstellungstechnischen Aufwand her vorteilhaft ist.

Die Stärke 108 der Platte (siehe Fig. 2) liegt grundsätzlich im Bereich von 0.5 mm bis 1.6 mm, bevorzugt liegt sie im Bereich von 0.8 m bis 1.2 mm, wie dies für sogenannten Miniplatten typisch ist, und kann ganz speziell etwa 1.0 mm betragen.

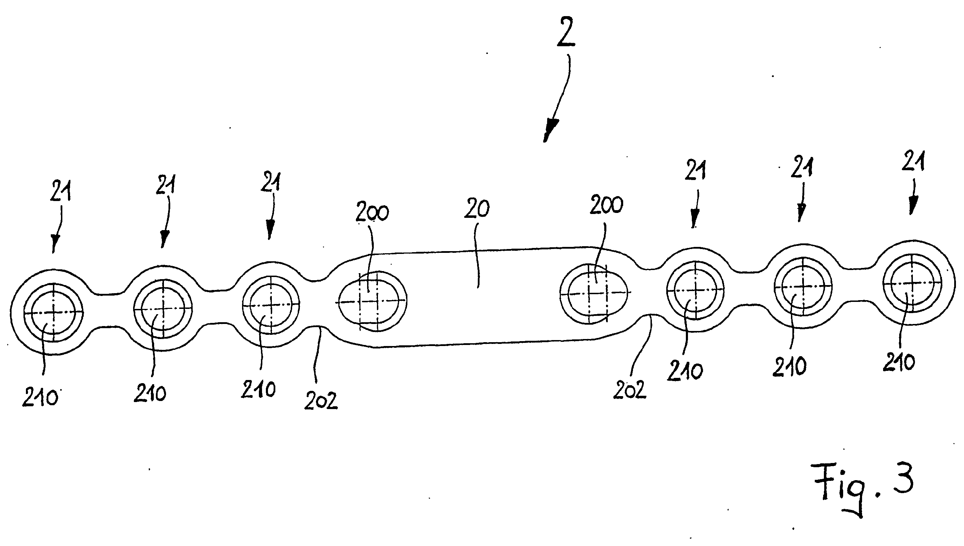

In Fig. 3 ist ein zweites Ausführungsbeispiel einer erfindungsgemässen Knochenplatte 2 in Aufsicht dargestellt. Die in Fig. 3 gezeigte Knochenplatte 2 weist einen Steg 20 auf, der in seinen Randregionen jeweils ein Kompressionsloch 200 aufweist, jedoch anders als bei der zuvor erläuterten Knochenplatte keine Durchgangslöcher und auch keine Schlitze hat. Stattdessen weist die Knochenplatte 2 jeweils drei Ösen 21 auf, wobei zwischen der am nächsten zum jeweiligen Kompressionsloch 200 gelegenen Öse 210 ein zwar taillierter, aber im Vergleich zu den Stegen zwischen den übrigen Ösen 210 verbreiterter Steg 202 vorgesehen ist. Dieser Steg 202 erlaubt ebenfalls bereits ein gewisses Anbiegen der Knochenplatte in diesem Bereich, weist aber andererseits noch eine erhöhte Stabilität gegen ein Verbiegen der Knochenplatte in der Plattenebene auf (sodass dieser Steg 202 funktionell in gewisser Weise mit dem Bereich mit dem Schlitz 102 des Ausführungsbeispiels gemäss Fig. 1 vergleichbar ist). Bezüglich der Ösenlöcher 210 gelten ansonsten die gleichen Ausführungen, die bereits weiter oben dargelegt sind.

Der Steg 20 ist erneut dazu bestimmt, über dem Frakturspalt zu liegen, die Enden der Knochenfragmente können mittels Kompressionsosteosynthese im Bereich des Stegs 20 stabil immobilisiert werden. Im Bereich der Ösen 21 bzw. der zwischen den Ösen 21 liegenden schmalen Stege kann die Knochenplatte 2 jeweils gut an die Knochenfragmente angebogen werden.

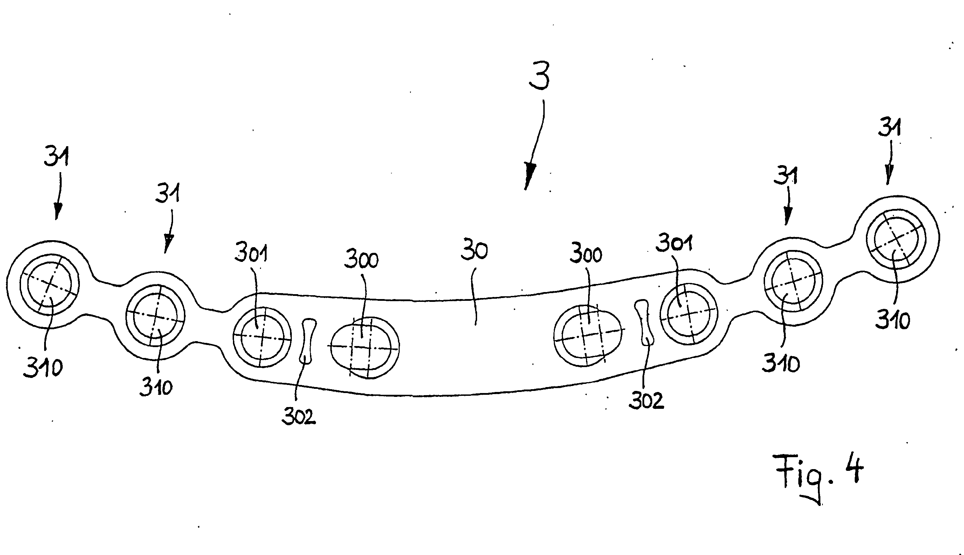

In Fig. 4 ist ein drittes Ausführungsbeispiel der erfindungsgemässen Knochenplatte 3 in Aufsicht dargestellt. Bei diesem Ausführungsbeispiel handelt es sich um eine anatomisch vorgeformte Knochenplatte, die Plattenlängsachse beschreibt einen leichten Bogen. Die Knochenplatte 3 ist aufgrund ihrer Form besonders geeignet für den Einsatz im Frontbereich des Unterkiefers.

Ansonsten entspricht die Knochenplatte 3 im wesentlichen der Knochenplatte 1, wie sie anhand von Fig. 1 und Fig. 2 beschrieben ist, weshalb bezüglich des Stegs 30, der Kompressionslöcher 300, der Durchgangslöcher 301 im Steg 30, der Schlitze 302, sowie bezüglich der Ösen 31 mit den Ösenlöchern 310 auf die entsprechenden Teile der Beschreibung der Knochenplatte 1 verwiesen werden kann.

## Patentansprüche

1. Knochenplatte (1;2;3) zur Versorgung einer Fraktur, insbesondere einer Fraktur eines atrophischen Unterkiefers, welche Knochenplatte (1;2;3) mehrere Ösen (11;21;31) aufweist, durch deren Löcher (110;210;310) hindurch Schrauben in die Knochenfragmente eingeschraubt werden können, um die Knochenplatte (1;2;3) an den Knochenfragmenten zu befestigen, sodass die Knochenfragmente diesseits und jenseits der Fraktur mit Hilfe der Knochenplatte (1;2;3) in einer gewünschten Position relativ zueinander gehalten werden, wobei die Knochenplatte (1;2;3) eine Plattenstärke (108) aufweist, die im Bereich von 0.5 mm bis 1.6 mm liegt, und ferner einen Stabilisierungsbereich (10;20;30) umfasst, der über der Fraktur zu liegen bestimmt ist, wobei zu beiden Seiten dieses Stabilisierungsbereichs (10;20;30) die Ösen (11;21;31) angeordnet sind, durch deren Löcher (110;210;310) hindurch die Schrauben in die Knochenfragmente geschraubt werden können, **dadurch gekennzeichnet, dass** die Knochenplatte (1;2;3) insgesamt einstreifig ausgebildet ist und der Stabilisierungsbereich als einstreifiger Steg (10;20;30) ausgebildet ist, dessen Breite (106,107) im wesentlichen dem Aussendurchmesser der Ösen (11;21;31) entspricht, wobei das Widerstandsmoment des Stegs (10;20;30) gegen ein Verbiegen der Knochenplatte in der Plattenebene gleich gross oder grösser ist als das entsprechende Widerstandsmoment der Knochenplatte im Bereich zwischen den Ösen (11;21;31).

2. Knochenplatte nach Anspruch 1, **dadurch gekennzeichnet, dass** die Plattenstärke (108) im Bereich von 0.8 mm bis 1.2 mm liegt.

3. Knochenplatte nach einem der Ansprüche 1 oder 2, **dadurch gekennzeichnet, dass** die Länge (104) des Stegs bis zu etwa sechs mal dem Abstand der Ösen (11;21;31)

4. Knochenplatte nach einem der Ansprüche 1 bis 3, **dadurch gekennzeichnet, dass** die Breite des Stegs (10;20;30) von der Mitte des Stegs ausgehend (106) zu den Randregionen des Stegs hin (107) abnimmt.

5. Knochenplatte nach Anspruch 3, **dadurch gekennzeichnet, dass** die Aussenkontur des Stegs (10;20;30) oval ist.

6. Knochenplatte nach einem der vorangehenden Ansprüche, **dadurch gekennzeichnet, dass** in den Randregionen des Stegs (10;20;30) Löcher (100,101;200,201;300,301) vorgesehen sind, durch welche hindurch Schrauben in die Knochenfragmente geschraubt werden können.

7. Knochenplatte nach Anspruch 6, **dadurch gekennzeichnet, dass** die Löcher in den Randregionen des Stegs (10;20;30) als Kompressionslöcher (100;200;300) ausgebildet sind.

8. Knochenplatte nach einem der vorangehenden Ansprüche, **dadurch gekennzeichnet, dass** in den Randregionen des Stegs (10;30) Schlitze (102;302) vorgesehen sind, um das Anbiegen der Knochenplatte (1;3) in diesen Randregionen des Stegs (10;30) zu erleichtern.

9. Knochenplatte nach einem der vorangehenden Ansprüche, **dadurch gekennzeichnet, dass** in den Löchern (110;210;310) der Ösen (11;21;31) und/oder in den Löchern (100,101;200,201;300,301) in den Randregionen des Stegs (10;20;30) eine Eingriffskontur vorgesehen ist zum winkelstabilen Verblocken der Knochenplatte (1;2;3) mittels eines an der Schraube vorgesehenen Verblockungsgewindes, wobei die Eingriffskontur ein Einschrauben der Schraube unter verschiedenen Winkeln zulässt.

## Claims

1. A bone plate (1; 2; 3) for treating a fracture, especially a fracture of an atrophic mandible, which bone plate (1; 2; 3) comprises a plurality of eyelets (11; 21; 31) through the holes (110; 210; 310) of which screws can be screwed into the bone fragments in order to fasten the bone plate (1; 2; 3) to the bone fragments so that the bone fragments on one side and on the other side of the fracture are maintained in a desired position relative to one another by means of the bone plate (1; 2; 3), said bone plate (1; 2; 3) having a plate thickness (108) in the range of from 0.5 mm to 1.6 mm, and moreover comprising a stabilization zone (10; 20; 30) intended to lie above the fracture, and the eyelets (11; 21; 31) through the holes (110; 210; 310) of which the screws can be screwed into the bone fragments are arranged on both sides of said stabilization zone (10; 20; 30), **characterized in that** the bone plate (1; 2; 3) as a whole is designed as a single strip, and the stabilization zone is configured as a strip web (10; 20; 30) whose width (106, 107) corresponds substantially to the external diameter of the eyelets (11; 21; 31), and the moment of resistance of the web (10; 20; 30) against bending of the bone plate in the plane of the plate is the same as or greater than the corresponding moment of resistance of the bone plate in the area between the eyelets (11; 21; 31).

2. The bone plate as claimed in claim 1, **characterized in that** the plate thickness (108) lies in the range of from 0.8 mm to 1.2 mm.

3. The bone plate as claimed in claim 1 or 2, **characterized in that** the length (104) of the web is up to about six times the distance of the eyelets (11; 21; 31) from one another.

4. The bone plate as claimed in any one of claims 1 through 3, **characterized in that** the width of the web (10; 20; 30) decreases from the middle of the web (106) to the edge regions (107) of the web.

5. The bone plate as claimed in claim 3, **characterized in that** the outer contour of the web (10; 20; 30) is oval.

6. The bone plate as claimed in any one of the preceding claims, **characterized in that**, in the edge regions of the web (10; 20; 30), holes (100, 101; 200, 201; 300, 301) are provided through which screws can be screwed into the bone fragments.

7. The bone plate as claimed in claim 6, **characterized in that** the holes in the edge regions of the web (10; 20; 30) are designed as compression holes (100; 200; 300).

8. The bone plate as claimed in any one of the preceding claims, **characterized in that**, in the edge regions of the web (10; 30), slits (102; 302) are provided to facilitate bending of the bone plate (1; 3) in these edge regions of the web (10; 30).

9. The bone plate as claimed in any one of the preceding claims, **characterized in that**, in the holes (110; 210; 310) of the eyelets (11; 21; 31) and/or in the holes (100, 101; 200, 201; 300, 301) in the edge regions of the web (10; 20; 30), an engagement contour is provided for blocking the bone plate (1; 2; 3) at a stable angle by means of a blocking thread provided on the screw, said engagement contour allowing the screw to be screwed in at different angles.

## Revendications

1. Plaque d'ostéosynthèse (1; 2; 3) pour le traitement d'une fracture, en particulier une fracture d'une mâchoire inférieure atrophiée, laquelle plaque d'ostéosynthèse (1; 2; 3) présente plusieurs oeillets (11; 21; 31) par les trous (110; 210; 310) desquels des vis peuvent être vissées dans les fragments d'os pour fixer la plaque d'ostéosynthèse (1; 2; 3) sur les fragments d'os de telle sorte que les fragments d'os soient maintenus dans une position relative souhaitée les uns par rapport aux autres d'un côté et de l'autre de la fracture à l'aide de la plaque d'ostéosynthèse (1; 2; 3), la plaque d'ostéosynthèse (1; 2; 3) présentant une épaisseur (108) comprise dans la plage de 0.5 mm à 1.6 mm et comportant en outre une zone de stabilisation (10; 20; 30) destinée à venir se placer au-dessus de la fracture, les oeillets (11; 21; 31) par les trous (110; 210; 310) desquels les vis peuvent être vissées dans les fragments d'os étant disposés des deux côtés de cette zone de stabilisation (10; 20; 30), **caractérisée en ce que** la plaque d'ostéosynthèse (1; 2; 3) présente une configuration globale en une bande, la zone de stabilisation étant configurée comme aile (10; 20; 30) en une seule bande dont la largeur (106, 107) correspond essentiellement au diamètre extérieur des oeillets (11; 21; 31), le couple résistant de l'aile (10; 20; 30) qui s'oppose à une flexion de la plaque d'ostéosynthèse dans le plan de la plaque étant égal ou. supérieur au couple résistant correspondant de la plaque d'ostéosynthèse dans la zone située entre les oeillets (11; 21; 31).

2. Plaque d'ostéosynthèse selon la revendication 1, **caractérisée en ce que** l'épaisseur (108) de la plaque est comprise dans la plage de 0.8 mm à 1.2 mm.

3. Plaque d'ostéosynthèse selon l'une des revendications 1 ou 2, **caractérisée en ce que** la longueur (104) de l'aile représente jusqu'environ six fois la distance entre les oeillets (11; 21; 31).

4. Plaque d'ostéosynthèse selon l'une des revendications 1 à 3, **caractérisée en ce que** la largeur de l'aile (10; 20; 30) diminue depuis le milieu de l'aile (106) jusqu'aux régions de bord de l'aile (107).

5. Plaque d'ostéosynthèse selon la revendication 3, **caractérisée en ce que** le contour extérieur de l'aile (10; 20; 30) est ovale.

6. Plaque d'ostéosynthèse selon l'une des revendications précédentes, **caractérisée en ce que** des trous (100, 101; 200, 201; 300, 301) par lesquels des vis peuvent être vissées dans fragments d'os sont prévus dans les zones de bord de l'aile (10; 20; 30).

7. Plaque d'ostéosynthèse selon la revendication 6, **caractérisée en ce que** les trous situés dans les zones de bord de l'aile (10; 20; 30) sont configurés comme trous de compression (100; 200; 300).

8. Plaque d'ostéosynthèse selon l'une des revendications précédentes, **caractérisée en ce que** des fentes (102; 302) sont prévues dans les zones de bord de l'aile (10; 30) pour faciliter la flexion de la plaque d'ostéosynthèse (1; 3) dans ces régions de bord de l'aile (10; 30).

9. Plaque d'ostéosynthèse selon l'une des revendications précédentes, **caractérisée en ce qu'**un contour d'engagement est prévu dans les trous (110; 210; 310) des oeillets (11; 21; 31) et/ou dans les trous (100, 101; 200, 201; 300, 301) situés dans les zones de bord de l'aile (10; 20; 30) pour bloquer la plaque d'ostéosynthèse (1; 2; 3) sous un angle fixe au moyen d'un filet de blocage prévu sur la vis, le contour d'engagement permettant de visser la vis sous différents angles.
